(19) Europäisches Patentamt
European Patent Office
Office européen des brevets

(11) Publication number: **0 497 200 A1**

(12) **EUROPEAN PATENT APPLICATION**

(21) Application number: **92100989.0**

(51) Int. Cl.5: **C07D 409/12**, A61K 31/47

(22) Date of filing: **22.01.92**

(30) Priority: **01.02.91 IT 25291**

(43) Date of publication of application: **05.08.92 Bulletin 92/32**

(84) Designated Contracting States: **AT BE CH DE DK ES FR GB GR IT LI LU MC NL PT SE**

(71) Applicant: **LA.FA.RE. S.r.l.**
**Via Sac. Benedetto Cozzolino, 77**
**I-80056 Ercolano (Napoli)(IT)**

(72) Inventor: **Marfe', Paolo**
**Via Sac. Benedetto Cozzolino, 77**
**I-80056 Ercolano (Napoli)(IT)**
Inventor: **Di Schiena, Michele**
**Via Garibaldi**
**I-20080 Cisliano (Milano)(IT)**

(74) Representative: **Minoja, Fabrizio**
**Studio Consulenza Brevettuale Via Rossini, 8**
**I-20122 Milano(IT)**

(54) **Ouinolone derivatives having antibacterial and mucolytic activities.**

(57) Compounds of formula (I)

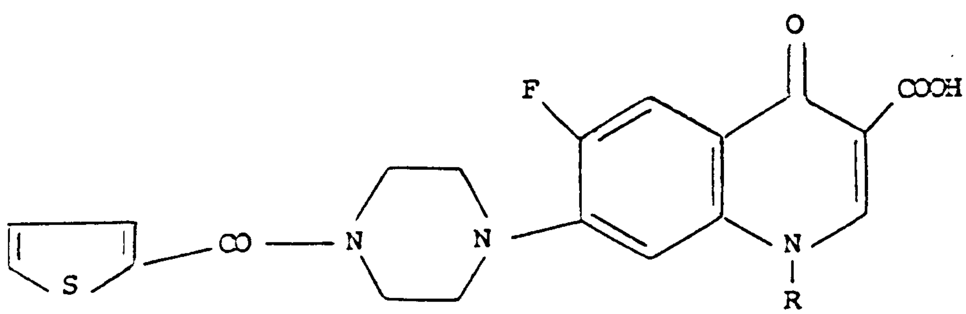

I

wherein R is $C_1$-$C_6$ alkyl or $C_3$-$C_7$ cycloalkyl and the pharmaceutically acceptable salts thereof, are useful in human therapy as mucolytic and antibacterial agents.

EP 0 497 200 A1

The present invention relates to compounds of formula I

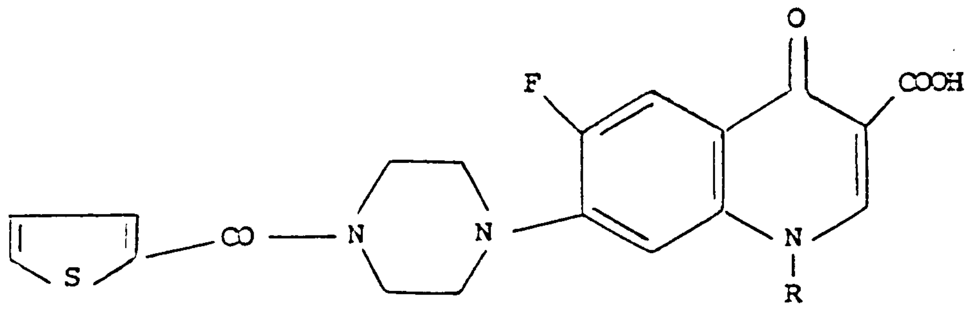

I

wherein R is $C_1$-$C_6$ alkyl or $C_3$-$C_7$ cycloalkyl and pharmaceutically acceptable salts thereof.

The invention also relates to pharmaceutical compositions containing as the active ingredient the compounds of formula (I) or the salts thereof with non-toxic acids or possible hydrate forms thereof.

According to the invention R is preferably ethyl or cyclopropyl. A particularly preferred compound is the one in which R is ethyl.

Compounds (I) can be prepared by reacting compounds of formula (II)

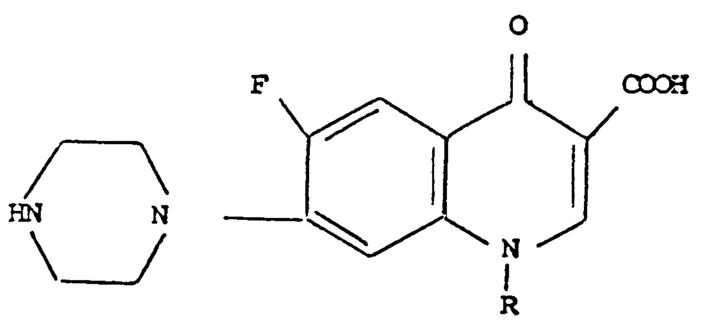

II

wherein R is as defined above, with a 2-thenoic acid reactive derivative, such as acid chloride, mixed or symmetrical anhydrides, imidazolide, reactive esters and the like, according to conventional methods.

Compounds (II) are known antibacterial agents which are disclosed in DE 2804097 and DE 3142854 and have already been used in human therapy, particularly for the treatment of infections of the urinary tract.

Compounds (I) have advantageous pharmacological properties. Particularly, compounds (I) have a wide spectrum antibacterial activity together with secretolytic and mucus regulating activities which give them a marked pulmonary tropism. Moreover, the compounds of the invention show a higher bioavailability than the known quinolone antibacterials, so that higher and longer-lasting therapeutically effective hematic and urinary concentrations are attained.

Therefore, the compounds of the present invention can be used as active ingredients in pharmaceutical compositions having antibacterial and mucus regulating activities for the treatment of the infections of urinary, bronchopulmonar, gastrointestinal systems, as well as of skin and the adnexa thereof, ear, nose, throat and generally of all the infections caused by gram-positive or gram-negative bacteria.

Therefore, the compositions of the invention will be used for the therapy of all those infections characterized by qualitative and quantitative changes in the exudate, such as bronchitis, bronchopneumonia, pleurisy, pharyngitis, thanks to the marked mucus regulating action qualitatively and quantitatively exerted.

The compositions of the invention can be prepared according to conventional techniques and excipients, such as those described in Remington's Pharmaceutical Sciences Handbook, Mack Pub. Co., NY, USA.

Said compositions can be administered by the oral, parenteral, topical and rectal routes. Therefore, suitable formulations will include capsules, tablets, syrups, vials, creams, suppositories and the like.

The dosage will depend on the pathology to be treated, as well as on the age, weight and conditions of the patient; generally the dosage will range from 5 to 50 mg/kg/day.

The following examples illustrate the invention.

## EXAMPLE 1

64 g of 1-ethyl-6-fluoro-1,4-dihydro-4-oxo-7-(1-piperazinyl) 3-quinolinecarboxylic acid, are suspended in a solution of 30 g of sodium carbonate in 1000 ml of distilled water. 1000 ml of acetone are added to the stirred suspension to obtain a nearly clear solution. Thereafter, 29 g of 2-thenoyl chloride (min. titre 98%) dispersed in 30 ml of acetone are dropped therein, under strong stirring, at room temperature. When the addition of chloride is completed, the resulting thick white suspension is stirred for about 16 hours, then it is filtered under reduced pressure and mother liquors are accurately discarded. The white solid is washed on the filter with portions of a mixture of: distilled water 200 ml, sodium carbonate 6 g, acetone 200 ml. Finally it is thoroughly washed with distilled water until the washing liquor has an almost neutral pH.

The washed solid is suspended in 1000 ml of a 10% acetic acid aqueous solution; the pH of the suspension must be lower than 2.5. The suspension is kept under stirring for at least 4 hours, then it is filtered under reduced pressure, washing the solid with distilled water until the washing liquor has neutral pH. The solid is dried at 60 ° C in air stream or under reduced pressure. A white microcrystalline solid is obtained; average weight yield 80 g.

This solid is dissolved in about 800 ml of anhydrous dimethylformamide at about 80 ° C. The suspension is quickly filtered through paper to removed any impurities, then filter is washed with a few warm dimethylformamide. The filtered solution is left to crystallize under slow stirring to room temperature, then it is cooled to 0/ + 5 ° C and it is left to crystallize for about 5 hours. The crystalline solid is filtered and washed with a few iced dimethylformamide, then with acetone to remove any dimethylformamide traces. The solid is dried under reduced pressure at about 60 ° C, to obtain 64 g of 1-ethyl-6-fluoro-1,4-dihydro-4-oxo-7-[4-(2-thenoyl)-piperazin-1-yl]-3-quinoline-carboxylic acid as a very white crystalline solid.

Analytical characteristics

TLC on silica gel F 254 Merck plate, eluent: ethyl acetate 50, acetone 20, glacial acetic acid 10, water 10 mixture, developer UV light 254: unitary spot with Rf about 0.7.
Melting point: 273-275 ° C (Gallenkamp apparatus).
NMR (DMSO)δ: 1.4 (t, 3H); 3.39 (m, 4H); 3.85 (m, 4H); 4.57 (q, 2H); 7.15 (dd, 1H); 7.19 (d, 1H); 7.49 (dd, 1H); 7.78 (dd, 1H); 7.78 (dd, 1H); 7.90 (d, 1H); 8.94 (s, 1H); 15.3 (O, 1H).

**EXAMPLE 2**

Capsules for the oral administration

Each animal gelatin capsule contains:

| | | |
|---|---|---|
| Active ingredient of example 1 | | 250 mg |
| Excipients: | lactose<br>magnesium stearate<br>hydroxypropylcellulose | 100 mg<br>4 mg<br>66 mg |

**Claims**
**Claims for the following Contracting States: AT, BE, CH, DE, FR, GB, IT, LI, LU, NL, SE, DK, MC**

**1.** Compounds of formula (I)

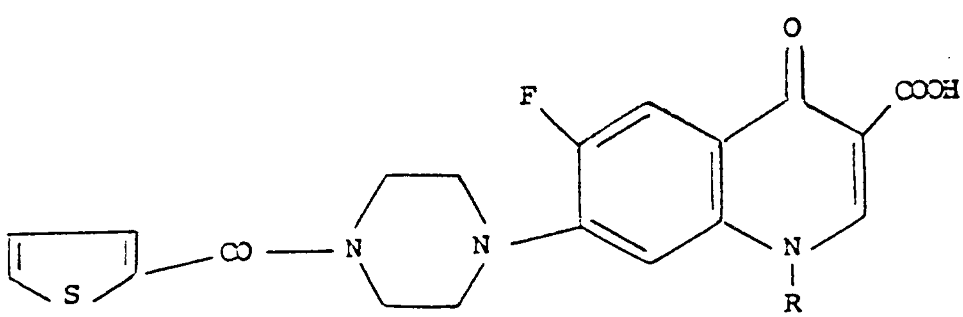

I

wherein R is $C_1$-$C_6$ alkyl or $C_3$-$C_7$ cycloalkyl and the pharmaceutically acceptable salts thereof.

**2.** A compound according to claim 1 in which R is ethyl or cyclopropyl.

**3.** Pharmaceutical compositions containing as the active ingredient the compounds of claims 1 or 2 in admixture with suitable carriers or excipients.

**4.** The use of the compounds of claims 1-2 for the preparation of a medicament having antibacterial, secretolytic and mucus regulating activities.

**Claims for the following Contracting States : ES, GR, PT.**

**1.** A process for the preparation of the compounds of formula (I)

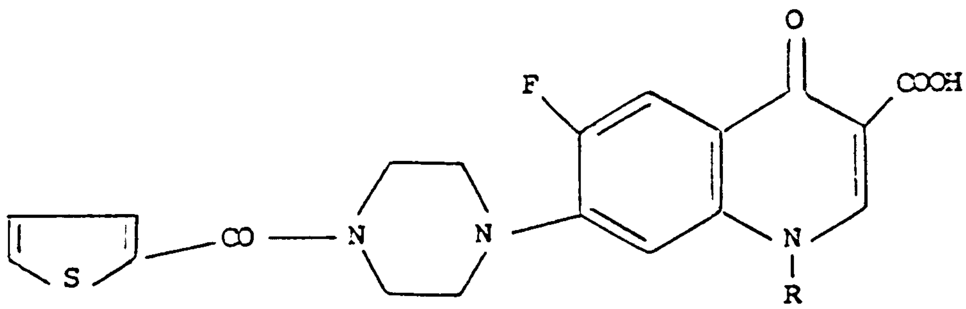

I

wherein R is $C_1$-$C_6$ alkyl or $C_3$-$C_7$ cycloalkyl, characterized in that compounds of formula (II)

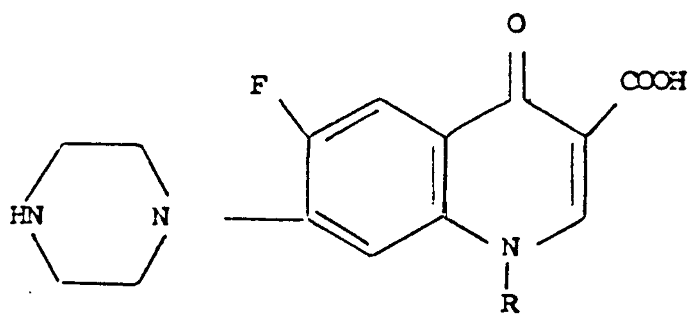

II

wherein R is as defined above, are reacted with a 2-thenoic acid reactive derivative.

**2.** A process according to claim 1, wherein the 2-thenoic acid reactive derivative is selected from the group consisting of acid chloride, mixed or symmetrical anhydrides, imidazolide, reactive esters.

European Patent Office

# EUROPEAN SEARCH REPORT

Application Number

EP 92 10 0989

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | CHEMICAL ABSTRACTS, vol. 115, 1991, Columbus, Ohio, US; abstract no. 71541F, H. C. CHEN ET AL.: 'Syntheses of 1-ethyl-6-fluoro-1,4-dihydro-4-oxo-7-((4-p-nitrobenzoyl)-1-piperazinyl)quinoline-3-carboxylic acid and its analogs.' page 791 ; & Yaoxue Xuebao 1991, 26(4), 299-302 * RN 135038-38-9 * * abstract * | 1 | C07D409/12 A61K31/47 |
| A | DE-A-3 542 002 (BAYER AG) * example 9; claims * | 1-3 | |
| A | US-A-4 762 845 (ABBOTT LABORATORIES) * example 6 * | 1,3 | |
| A | EP-A-0 230 053 (AMERICAN CYANAMID COMPANY) * example 26,30,31,60 etc. * | 1,3 | |

TECHNICAL FIELDS SEARCHED (Int. Cl.5 )

C07D

The present search report has been drawn up for all claims

| Place of search | Date of completion of the search | Examiner |
|---|---|---|
| THE HAGUE | 23 MARCH 1992 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document